(19) Europäisches Patentamt
European Patent Office
Office européen des brevets

(11) Veröffentlichungsnummer: **0 218 123 B1**

# (12) EUROPÄISCHE PATENTSCHRIFT

(45) Veröffentlichungstag der Patentschrift: **22.03.89**

(51) Int. Cl.[4]: **C 07 C 131/00**

(21) Anmeldenummer: **86112754.6**

(22) Anmeldetag: **16.09.86**

## (54) Verfahren zur Herstellung von Hydroxy-benzaldoxim-O-ethern.

(30) Priorität: **28.09.85 DE 3534731**

(43) Veröffentlichungstag der Anmeldung: **15.04.87 Patentblatt 87/16**

(45) Bekanntmachung des Hinweises auf die Patenterteilung: **22.03.89 Patentblatt 89/12**

(84) Benannte Vertragsstaaten: **AT BE CH DE FR GB IT LI NL**

(56) Entgegenhaltungen:
**GB-A- 870 787**

**THE JOURNAL OF THE AMERICAN CHEMICAL SOCIETY, Band 78, 1956, Seite 2470; Washington, D.C., US; M.S. NEWMAN et al.: "Alpha-(2,4,5,7-Tetranitro-9-fluorenylideneaminoöxy)-propionic Acid, a New Reagent for Resolution by Complex Formation"**

(73) Patentinhaber: **BAYER AG, Konzernverwaltung RP Patentabteilung, D-5090 Leverkusen 1 Bayerwerk (DE)**

(72) Erfinder: **Elbe, Hans-Ludwig, Dr., Dasnöckel 59, D-5600 Wuppertal 1 (DE)**

EP 0 218 123 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von bekannten Hydroxybenzaldoxim-O--ethern, die als Zwischenprodukte zur Synthese von Verbindungen mit fungizider, insektizider und antimykotischer Wirksamkeit verwendet werden können.

Es ist bereit bekannt geworden, daß man bestimmte Hydroxy-benzaldoxim-O-ether herstellen kann, indem man Hydroxybenzaldehyde mit den entsprechenden Hydroxylamin-Derivaten umsetzt (vgl. EP-OS 0 076 370 und EP-OS 0 115 828). Die betreffende Umsetzung läßt sich durch das folgende Reaktionsschema veranschaulichen:

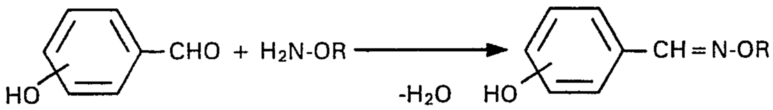


R = Alkyl, Alkenyl, Alkinyl.

Nachteilig an diesem Verfahren ist jedoch, daß reine Hydroxylamin-Derivate oder deren Salze benötigt werden. Diese sind aber so teure Ausgangsstoffe, daß ihr Einsatz für eine Herstellung von Hydroxy--benzaldoxim-O-ethern in technischem Maßstab uninteressant ist. Ungünstig ist auch, daß üblicherweise des teure Hydroxylamin-Derivat im Überschuß eingesetzt wird, um einen möglichst vollständigen Umsatz des Aldehyds zu erzielen.

Weiterhin ist bekannt, daß sich benzaldoxim-O--ether, die keine Hydroxy-Gruppe im Phenyl-Teil enthalten, dadurch herstellen lassen, daß man Benzaldoxim in Gegenwart von Basen mit Alkyl-, Alkenyl- oder Alkinyl-halogeniden umsetzt (vgl. Houben-Weyl, «Methoden der Organischen Chemie», Band 10/4, Seiten 217-223, Georg Thieme Verlag, Stuttgart, 1971). Die betreffende Umsetzung kann durch das folgende Reaktionsschema veranschaulicht werden:

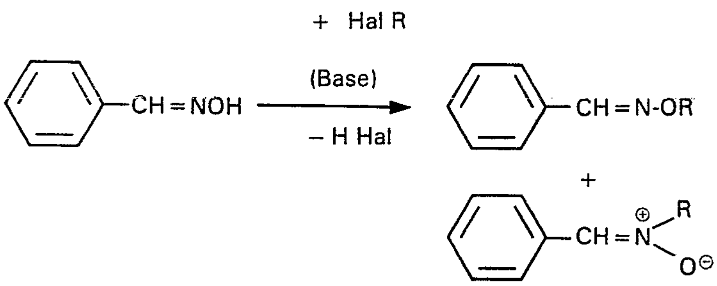


R = Alkyl, Alkenyl, Alkinyl
Hal = Halogen.

Dieses Verfahren hat den Nachteil, daß es stets zu Gemischen von Benzaldoxim-O-ethern und unerwünschten Nitronen führt. Der Anteil an Oxim-ether kann zwar dadurch erhöht werden, daß man das als Ausgangsprodukt verwendete Benzaldoxim in Form eines Salzes, — zum Beispiel in Form des Silbersalzes, einsetzt, jedoch stellt diese Variante keine technisch akzeptable Alternative dar.

Die Alkylierung von syn-Benzaldoxim-Natriumsalz mit Alkylhalogeniden in alkoholischer Lösung liefert die O-Alkylether in Ausbeuten von 50 bis 80%. Unter gleichen Bedingungen erfolgt aber beim Einsatz von anti-Benzaldoxim ausschließlich eine N-Alkylierung zum Nitron [vgl. J. Org. Chem. 32, 261 (1967)].

Entsprechende Reaktionen sind auch unter Phasentransfer-Bedingungen beschrieben (vgl. Chemistry Letters, 1980, S. 869-870). So liefert die Butylierung von syn-Benzaldoxim in einer phasentransferkatalysierten Reaktion (Methylenchlorid, wäßrige Natronlauge, Tetrabuylammoniumbromid) den syn-Bentaldoxim-O-butylether in einer Ausbeute von 58%, während beim Einsatz von anti-Benzaldoxim das entsprechende Nitron das Hauptprodukt ist.

Es ist ferner bereits ein technisches Verfahren zur Herstellung O-substituierter Oxime von Ketonen beschrieben, bei dem Alkalisalze von Ketoximen, bzw. Keloxime in Gegenwart von festen Alkalihydroxiden, mit Alkylierungsmitteln in dipolar-aprotischen Lösungsmitteln umgesetzt werden. Zur Aufarbeitung wird mit Wasser versetzt und das Reaktionsprodukt extrahiert (vgl. EP-OS 0 023 560).

Die dabei vorzugsweise verwendeten Lösungsmittel, wie N-Methylpyrrolidon, Dimethylacetamid, Dimethylsulfoxid, Tetramethylensulfon oder Hexamethylphosphorsäuretriamid, stellen teure und teilweise toxikologisch bedenkliche Solventien dar, deren Wiedergewinnung aufgrund ihrer guten Wasserlöslichkeit und des hohen Siedepunktes problematisch ist. Der Wert dieses Verfahren ist somit, insbesondere im Hinblick auf eine technische Anwendung, stark eingeschränkt.

Weiterhin ist bekannt, daß O-substituierte Ketoxime und Aldoxime in einem technischen Prozeß erhalten werden können, wenn man Oxime mit einem Organochlorid und einem Alkalimetall-Hydroxid in einem wiedermolekularen Alkohol umsetzt (vgl. EP-OS 0 121 701). Die Ausbeuten an gewünschtem Oximeter sind jedoch unbefriedigend. So erhält man beispielsweise bei der Methylierung von Benzaldoxim ein Gemisch aus 72,9% O-Methylether, 21,6% Nitron und 4,7% Ausgangsprodukt (vgl. Beispiel 1 der EP-OS 0 121 701).

Für die Herstellung von 4-Hydroxy-benzaldoxim-O-ethern im technischen Maßstab stellt keines der bekannten Verfahren eine geeignete Möglichkeit dar.

Das technische Verfahren gemäß EP-OS 0 121 701, wie auch alle anderen aufgezählten generellen Alternativen sind insbesondere auch dadurch charackterisiert, daß die Alkylierung der Oxime unter basischen Bedingungen durchgeführt wird. Dieser Umstand schränkt die Anwendungsbreite der Verfahren auf solche Benzaldoxime ein, die keine basenempfundliche Substituenten im Aromaten tragen und/oder keine unter basischen Bedingungen durch das Alkylierungsmittel anzugreifende Substituenten tragen, wie beispielsweise phenolische OH-Gruppen.

Es wurde nun gefunden, daß man bekannte Hydroxy-benzaldoxim-O-ether der Formel

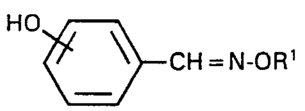

(I)

in welcher
$R^1$ für Alkyl, Alkenyl oder Alkinyl steht,

erhält, wenn man Hydroxy-benzaldehyde der Formel

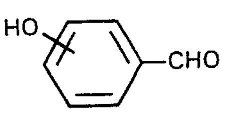

(II)

mit Ketoxim-O-ethern der Formel

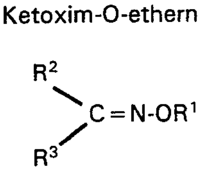

(III)

in welcher
$R^1$ die oben angegebene Bedeutung hat und
$R^2$ und $R^3$ gleich oder verschieden sind und für einen aliphatischen, cycloaliphatischen oder aromatischen Rest stehen, oder
$R^2$ und $R^3$ gemeinsam mit dem Kohlenstoffatom, an das sie gebunden sind, für Cycloalkyl stehen,

in Gegenwart eines sauren Katalysators und gegebenenfalls in Gegenwart eines Verdünnungsmittels bei Temperaturen zwischen 50°C und 150°C umsetzt.

Es ist als äußerst überraschend zu bezeichnen, daß die erfindungsgemäße Umsetzung unter den angegebenen Verfahrensbedingungen glatt abläuft. Aufgrund des bekannten Standes der Technik war zu erwarten, daß die gewünschte Umsetzung nur sehr langsam erfolgt, da bekannt ist, daß Ketoxim-ether gegen Säuren und Basen sehr widerstandsfähig sind. So gelingt z.B. die Verseifung erst bei längerem Erhitzen mit Mineralsäure (vgl. hierzu z.B. Houben-Weyl, Methoden der Organischen Chemie, Band X/1, Seite 1186, Stuttgart 1981; Helv. Chim. Acta, Vol. XLV, Fasc. 1 (1962) No 40, S. 359 sowie EP-OS 0 121 701).

Weiterhin war zu erwarten, daß das bei der Oximetherspaltung entstehende Keton ($R^2$-CO-$R^3$) unter saurer Katalyse mit dem Ausgangsaldehyd der Formel (II) in starkem Maß kondensieren würde, da bekannt ist, daß Hydroxy-Benzaldehyde in einer sauer katalysierten Aldol-Kondensation sehr leicht mit Ketonen, die eine zur Carbonylgruppe α-ständige Methylengruppe enthalten, zu α,β-ungesättigten Ketonen reagieren (vgl. hierzu Houben-Weyl, Methoden der Organischen Chemie, Band VII/2b, S. 1457, Stuttgart 1976).

Schließlich war zu erwarten, daß die erfindungsgemäße Umsetzung in einem ungünstigen Gleichgewichtsgemisch endet, wenn das entstehende Keton nicht weitgehend aus dem Reaktionsgemisch abgetrennt wird. Es ist nämlich bekannt, daß man Oxime schwerflüchtiger Ketone durch Umoximierung mit Oximen aus leichtflüchtigen Ketonen oder Aldehyden erhalten kann, wobei jedoch die entstehende leichtflüchtige Carbonylverbindung immer destillativ abgetrennt werden muß (vgl. BG-PS 870 787).

Im Fall der Oxime ist die destillative Entfernung des entstehenden Ketons wegen der hohen Siedepunktsdifferenzen von Keton bzw. Aldehyd und zugehörigem Oxim technisch einfach.

Die Trennung von Keton und zugehörigem Oximether beim erfindungsgemäßen Verfahren ist aber wegen der im allgemeinen geringen Siedepunktdifferenzen nur durch eine zeit- und energieaufwendige Feindestillation möglich.

Eine rasche Entfernung des in der Reaktion entstehenden Ketons $R^2$-CO-$R^3$ ist damit nicht möglich. Deshalb würde man eine in großem Ausmaß erfolgende Weiterreaktion des Ketons mit überschüssigem Hydroxy-benzaldehyd zu α,β-ungesättigten Ketonen erwarten.

Das erfindungsgemäße Verfahren zeichnet sich durch eine Reihe von Vorteilen aus. So ermöglicht es die Herstellung von Hydroxy-benzaldoxim-O-ethern der Formel (I) in hohen Ausbeuten, wobei billige und gut zugängliche Verbindungen als Ausgangsprodukte eingesetzt werden. Ferner ist die Umsetzung einfach durchführbar und die Isolierung der Benzaldoxim-O-ether der Formel (I) bereitet keinerlei Schwierigkeiten. Das erfindungsgemäße Verfahren ist daher besonders geeignet, um Hydroxy-benzaldoxim-O-ether der Formel (I) in technischem Maßstab herzustellen.

Verwendet man beispielsweise 4-Hydroxy-benzaldehyd und Methyl-isobutyl-Ketoxim-O-methylether als Ausgangsstoffe und konzentrierte Schwefelsäure als Katalysator, so kann der Verlauf des erfindungsgemäßen Verfahrens durch das folgende Formelschema veranschaulicht werden:

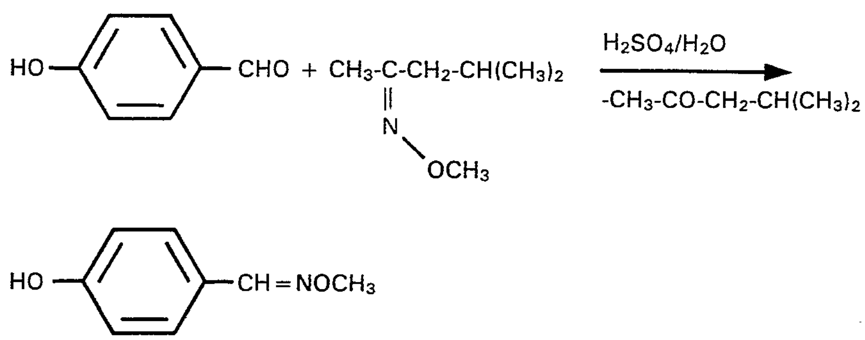


Die bei dem erfindungsgemäßen Verfahren als Ausgangsstoffe zu verwendenden Hydroxy-benzaldehyde sind durch die Formel (II) allgemein definiert. Besonders bevorzugt ist der 4-Hydroxy-benzaldehyd.

Die Hydroxybenzaldehyde der Formel (II) sind allgemein bekannte Verbindungen der organischen Chemie [vgl. z.B. Houben-Weyl, Methoden der Organischen Chemie, Band VII, Teil 1 und E 3 (1983)].

Die für das erfindungsgemäße Verfahren außerdem als Ausgangsstoffe zu verwendenden Ketoxim-O-ether sind durch die Formel (III) allgemein definiert. In dieser Formel stehen vorzugsweise

- $R^1$ für geradkettiges oder verzweigtes Alkyl mit 1 bis 10 Kohlenstoffatomen sowie für geradkettiges oder verzweigtes Alkenyl und Alkinyl mit jeweils 3 bis 10 Kohlenstoffatomen;
- $R^2$ für geradkettiges oder verzweigtes Alkyl mit 1 bis 10 Kohlenstoffatomen, Cycloalkyl mit 5 bis 7 Kohlenstoffatomen sowie für gegebenenfalls einfach oder zweifach, gleich oder verschieden substituiertes Phenyl, wobei als Substituenten vorzugsweise Halogen und Alkyl mit 1 oder 2 Kohlenstoffatomen genannt seien;
- $R^3$ für geradkettiges oder verzweigtes Alkyl mit 1 bis 10 Kohlenstoffatomen, Cycloalkyl mit 5 bis 7 Kohlenstoffatomen sowie für gegebenenfalls einfach oder zweifach, gleich oder verschieden substituiertes Phenyl, wobei als Substituenten vorzugsweise Halogen und Alkyl mit 1 oder 2 Kohlenstoffatomen genannt seien; sowie
- $R^2$ und $r^3$ gemeinsam mit dem Kohlenstoffatom, an das sie gebunden sind, für Cycloalkyl mit 5 bis 7 Kohlenstoffatomen.

Besonders bevorzugte Ausgangsstoffe sind diejenigen Ketoxim-O-ether der Formel (III), in denen

- $R^1$ für geradkettiges oder verzweigtes Alkyl mit 1 bis 4 Kohlenstoffatomen steht;
- $R^2$ für geradkettiges oder verzweigtes Alkyl mit 1 bis 5 Kohlenstoffatomen sowie für Phenyl steht;
- $R^3$ für geradkettiges oder verzweigtes Alkyl mit 1 bis 6 Kohlenstoffatomen sowie für Phenyl steht; oder
- $R^2$ und $R^3$ gemeinsam mit dem Kohlenstoffatom, an das sie gebunden sind, für Cyclohexyl oder Cycloheptyl stehen.

Die Ketoxim-O-ether der Formel (III) sind bekannt oder können in bekannter Art und Weise erhalten werden, indem man Ketoxime der Formel

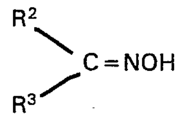 (IV)

in welcher

$R^2$ und $R^3$ die oben angegebene Bedeutung haben,

mit einem Alkylierungsmittel, wie beispielsweise Alkylhalogeniden oder Dimethylsulfat, in Gegenwart eines inerten organischen Lösungsmittels, wie beispielsweise Methylenchlorid, und in Gegenwart einer Base, wie beispielsweise Natronlauge, sowie in Gegenwart eines Phasentransferkatalysator, wie beispielsweise Tetrabutylammoniumbromid, umsetzt (vgl. hierzu z.B. EP-OS 0 023 560, EP-OS 0 121 701 sowie die Herstellungsbeispiele). Diejenigen Verbindungen der Formel (III), in denen $R^1$ für Alkenyl oder Alkinyl steht, lassen sich in analoger Weise herstellen.

Die Ketoxime der Formel (IV) sind allgemein bekannte Verbindungen der organischen Chemie. Sie lassen sich herstellen, indem man Ketone der Formel

$$R^2\text{-CO-}R^3 \qquad (V)$$

in welcher

$R^2$ und $R^3$ die oben angegebene Bedeutung haben,

nach üblichen Methoden mit Hydroxylamin oder dessen Salzen, gegebenenfalls in Gegenwart eines inerten organischen Verdünnungsmittels, umsetzt.

Dabei lassen sich die Oxime der Formel (IV) besonders vorteilhaft herstellen, wenn man eine technische Hydroxylaminsalz-Lösung mit dem Keton der Formel (V), gegebenenfalls in Gegenwart eines inerten, in Wasser schwer löslichen Lösungsmittels, wie beispielsweise Cyclohexan, Ligroin, Toluol oder Benzol, in einem pH-Bereich von 3 bis 7, vorzugsweise von 5 bis 6, umsetzt. Zur Aufarbeitung wird die organische Phase abgetrennt und fraktioniert destilliert. Dabei erhält man nach einem Vorlauf von überschüssigen Keton in hohen Ausbeuten die Oxime der Formel (IV) (vgl. auch die Herstellungsbeispiele).

Die Ketone der Formel (V) sind allgemeinen bekannte Verbindungen der organischen Chemie. Beispielsweise seien genannt:

Aceton, Methyl-isopropyl-keton, Methyl-n-propyl-keton, Methyl-isobutyl-keton, Methyl-ethyl-keton, tert.-Butyl-methyl-keton, Di-n-propyl-keton, Diiso-propyl-keton, Di-isobutyl-keton, Diethylketon, 5-Methyl-3-heptanon, Acetophenon, Cyclohexanon und Cycloheptanon.

Das erfindungsgemäße Verfahren wird gegebenenfalls in Gegenwart eines unter den Reaktionsbedingungen inerten organischen Lösungsmittels durchgeführt. Hierzu gehören vorzugsweise Alkohole, wie Methanol, Ethanol, n-Propanol, i-Propanol, n-Butanol oder tert.-Butanol; aliphatische und aromatische Kohlenwasserstoffe, wie Petrolether, Cyclohexan, Benzol oder Toluol; Ether wie Diisopropylether, Tetrahydrofuran oder Dioxan, sowie halogenierte Kohlenwasserstoffe, wie 1,2-Dichlorethan.

Als Katalysatoren kommen für das erfindungsgemäße Verfahren vorzugsweise Lewis-Säuren in Frage. Hierzu gehören vorzugsweise saure Salze organischer und anorganischer Säuren. Beispielhaft seien genannt: Salzsäure, Schwefelsäure, Phosphorsäure, Benzolsulfonsäure, Toluolsulfonsäure, Alkylcarbonsäuren, Alkylsulfonsäure, Trifluoressigsäure sowie fluorierte Alkylsulfonsäuren.

Die Reaktionstemperaturen können bei der Durchführung des erfindungsgemäßen Verfahrens in einem größeren Bereich variiert werden. Im allgemeinen arbeitet man bei Temperaturen zwischen 50 und 150°C, vorzugsweise zwischen 50 und 100°C.

Bei der Durchführung des erfindungsgemäßen Verfahrens setzt man auf 1 Mol Hydroxy-benzaldehyd der Formel (II) im allgemeinen 1 bis 10 Mol, vorzugsweise 1 bis 4 Mol Ketoxim-O-ether der Formel (III) und 0,01 bis 10 Mol-% Katalysator, bezogen auf eingesetzten Ketoxim-O-ether, ein.

Ein Überschuß Ketoxim-O-ether ist nicht unwirtschaftlich, da nicht umgesetzter Ketoxim-O-ether durch Destillation vom Reaktionsgemisch abgetrennt und recyclisiert werden kann.

Zur Aufarbeitung wird das Reaktionsgemisch mit Wasser versetzt, die organische Phase abgetrennt und fraktioniert destilliert. Es ist aber auch jede andere Aufarbeitungsvariante möglich. Beispielsweise kann es sich als vorteilhaft erweisen, das sich bildende Keton durch fraktionierte Destillation aus dem Reaktionsgemisch zu entfernen.

Die nach dem erfindungsgemäßen Verfahren herstellbaren Hydroxy-benzaldoxim-O-ether der Formel (I) sind allgemein wertvolle Ausgangsstoffe zur Synthese von biologisch aktiven Verbindungen, wie z.B. zur Synthese von Oximethern, welche gute insektizide Eigenschaften besitzen (vgl. EP-OS 0 115 828); von Azolyl-phenoxy-Derivaten, welche hervorragende fungizide Eigenschaften aufweisen (vgl. EP-OS 0 076 370); von 1-Hydroxyethyl-triazolyl-Derivaten, welche gute fungizide und antimykotische Eigenschaften aufweisen (vgl. EP-OS 0 110 048 und DE-OS 3 314 548); sowie von Hydroxyalkylazol-Derivaten, welche gute antimykotische Wirksamkeit besitzen (vgl. DE-OS 3 427 844).

So läßt sich beispielsweise das 3,3-Dimethyl-1-(4-methoximinomethyl-phenoxy)-1-(1,2,4-triazol-1-yl)-butan-2-on der Formel

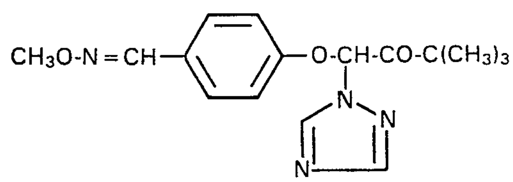

herstellen, indem man 3,3-Dimethyl-1-(1,2,4-triazol-1-yl)-butan-2-on zunächst mit Brom zum 1-Brom-1-(1,2,4-triazol-1-yl)-3,3-dimethyl-butan-2-on umsetzt und dieses anschließend mit 4-Hydroxybenzaldoxim-O-methylether in Gegenwart einer Base umsetzt. Diese Synthese lässt sich formelmäßig wie folgt veranschaulichen:

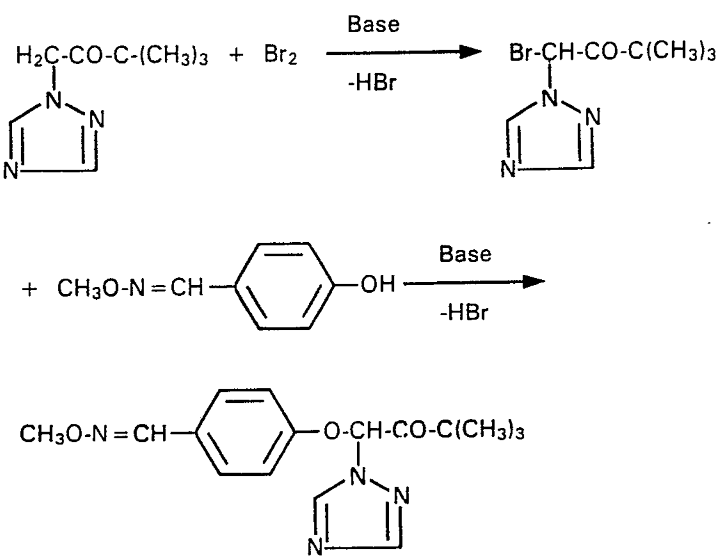

Das erfindungsgemäße Verfahren wird durch die nachfolgenden Beispiele veranschaulicht.

*Beispiel 1*

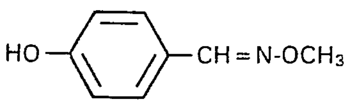

(I-1)

122 g (1 Mol) 4-Hydroxybenzaldehyd, 258 g (2 Mol) Methyl-isobutyl-ketoxim-O-methylether und 9,8 g (0,05 Mol) 50-Gew.-%ige Schwefelsäure werden 6 Stunden bei 100°C gerührt. Danach läßt man abkühlen, wäscht mit Wasser neutral und destilliert die organische Phase im Vakuum.

Nach einem Vorlauf von Methylisobutylketon und Methylisobutylketoxim-O-methylether erhält man 140,5 g (93% der Theorie) 4-Hydroxy-benzaldoxim-O-methylether vom Siedepunkt 138-142°C/ 0,1 Torr.

*Herstellung des Ausgangsproduktes*

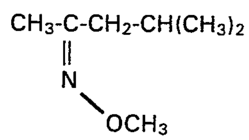

(III-1)

120 g (1,04 Mol) Methyl-isobutyl-ketoxim, 500 g (2,5 Mol) 20-Gew.-%ige Natronlauge und 33 g (0,104 Mol) Tetrabutylammoniumbromid werden in 500 ml Methylenchlorid vorgelegt. Bei 40°C läßt man 138 g (1,09 Mol) Dimethylsulfat zutropfen und das Reaktionsgemisch 7 Stunden bei 40°C nachrühren. Danach wird abgekühlt, die organische Phase abgetrennt, mit Wasser gewaschen, über Natriumsulfat getrocknet und fraktioniert destilliert. Man erhält 84 g (60% der Theorie) Methylisobutyl-ketoxim-O-methylether vom Siedeintervall 120 bis 129°C/760 Torr.

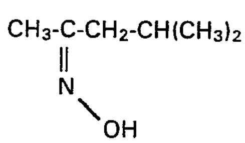

(IV-1)

2950 g (5 Mol) 14 Gew.-%ige technische Hydroxylaminsulfat-Lösung wird mit halbkonzentrierter Natronlauge auf einen pH-Wert von 5 bis 6 eingestellt. Bei 20-30°C gibt man 1000 g (10 Mol) Methylisobutyl-Keton zu und hält den pH-Wert im Verlauf der Reaktion durch weitere Zugabe von Natronlauge konstant. Nach 2 Stunden ist die Reaktion abgeschlossen. Die organische Phase wird abgetrennt, mit Wasser gewaschen, über Natriumsulfat getrocknet und destilliert. Man erhält 515 g (89,6% der Theorie, bezogen auf eingesetztes Hydroxylamin) Methylisobutyl-ketoxim vom Siedepunkt 88-91°C/ 35 Torr.

*Herstellung weiterer Ausgangsstoffe der Formel (III)*

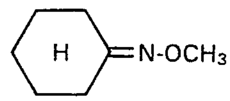

(III-2)

113 g (1 Mol) Cyclohexanon-oxim, 213 g (2,4 mol) 45 Gew.-%ige Natronlauge und 32 g (0,1 mol) Tetrabutylammoniumbromit werden in 500 ml Methylenchlorid vorgelegt. Man läßt bei 40°C 139 g (1,1 Mol) Dimethylsulfat zutropfen und rührt 7 Stunden bei 40°C nach. Anschließend wird mit Wasser versetzt. Die organische Phase wird abgetrennt, über Natriumsulfat getrocknet und fraktioniert destilliert.

Man erhält 70 g (55% der Theorie) Cyclohexanon-oxim-O-methylether vom Siedeintervall 160-164°C/ 760 Torr.

**Patentansprüche**

1. Verfahren zur Herstellung von Hydroxy-benzaldoxim-O-ethern der Formel

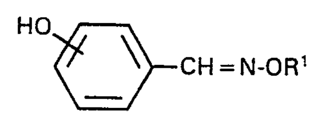

(I)

in welcher

$R^1$ für Alkyl, Alkenyl oder Alkinyl steht,

dadurch gekennzeichnet, daß man Hydroxybenzaldehyde der Formel

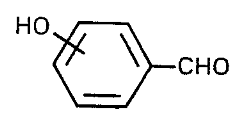

(II)

mit Ketoxim-O-ethern der Formel

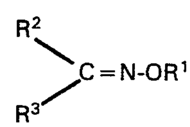

(III)

in welcher

$R^1$ die oben angegebene Bedeutung hat und
$R^2$ und $R^3$ gleich oder verschieden sind und für einen aliphatischen, cycloaliphatischen oder aromatischen Rest stehen, oder
$R^2$ und $R^3$ gemeinsam mit dem Kohlenstoffatom, an das sie gebunden sind, für Cycloalkyl stehen,

in Gegenwart eines sauren Katalysators und gegebenenfalls in Gegenwart eines Verdünnungsmittels bei Temperaturen zwischen 50 und 150°C umsetzt.

2. Verfahren gemäß Anspruch 1, dadurch gekennzeichnet, daß man als Ausgangsstoff der Formel (II) 4-Hydroxybenzaldehyd einsetzt.

3. Verfahren gemäß Anspruch 1, dadurch gekennzeichnet, daß man als Ausgangsstoffe Ketoxim-O-ether der Formel (III) einsetzt, in welcher

$R^1$ für geradkettiges oder verzweigtes Alkyl mit 1 bis 20 Kohlenstoffatomen sowie für geradkettiges oder verzweigtes Alkenyl und Alkinyl mit jeweils 3 bis 10 Kohlenstoffatomen steht,
$R^2$ für geradkettiges oder verzweigtes Alkyl mit 1 bis 10 Kohlenstoffatomen, Cycloalkyl mit 5 bis 7 Kohlenstoffatomen sowie für gegebenenfalls einfach oder zweifach, gleich oder verschieden durch Halogen und/oder Alkyl mit 1 oder 2 Kohlenstoffatomen substituiertes Phenyl steht,
$R^3$ für geradkettiges oder verzweigtes Alkyl mit 1 bis 10 Kohlenstoffatomen, Cycloalkyl mit 5 bis 7 Kohlenstoffatomen sowie für gegebenenfalls einfach oder zweifach, gleich oder verschieden durch Halogen und/oder Alkyl mit 1 bis 2 Kohlenstoffatomen substituiertes Phenyl steht, oder
$R^2$ und $R^3$ gemeinsam mit dem Kohlenstoffatom, an das gebunden sind, für Cycloalkyl mit 5 bis 7 Kohlenstoffatomen stehen.

4. Verfahren gemäß Anspruch 1, dadurch gekennzeichnet, daß man als saure Katalysatoren Lewis-Säuren einsetzt.

5. Verfahren gemäß Anspruch 1, dadurch gekennzeichnet, daß man die Umsetzung bei Temperaturen zwischen 50 und 100°C durchführt.

6. Verfahren gemäß Anspruch 1, dadurch gekennzeichnet, daß man auf 1 Mol Hydroxy-benzaldehyd der Formel (II) 1 bis 10 Mol Ketoxim-O-ether der Formel (III) und 0,01 bis 10 Mol-% Katalysator, bezogen auf Ketoxim-O-ether, einsetzt.

**Claims**

1. Process for the preparation of hydroxybenzaldoxime O-ethers of the formula

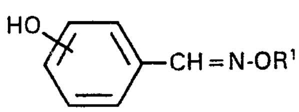 (I)

in which

$R^1$ represents alkyl, alkenyl or alkynyl,

characterized in that hydroxybenzaldekydes of the formula

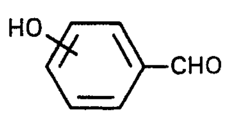 (II)

are reacted, in the presence of an acid catalyst and, where appropriate, in the presence of a diluent, at temperatures between 50 and 150°C, with ketoxime O-ethers of the formula

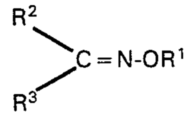 (III)

in which

$R^1$ has the abovementioned meaning, and
$R^2$ and $R^3$ are identical or different and represent an aliphatic, cycloaliphatic or aromatic radical, or
$R^2$ and $R^3$, together with the carbon atom to which they are bonded, represent cycloalkyl.

2. Process according to Claim 1, characterized in that 4-hydroxybenzaldehyde is used as the starting material of the formula (II).

3. Process according to Claim 1, characterized in that ketoxime O-ethers of the formula (III) in which

$R^1$ represents straight-chain or branched alkyl having 1 to 20 carbon atoms, and represents straight-chain or branched alkenyl and alkynyl, each having 3 to 10 carbon atoms,
$R^2$ represents straight-chain or branched alkyl having 1 to 10 carbon atoms, cycloalkyl having 5 to 7 carbon atoms, and represents phenyl which is optionally substituted once or twice, identically or differently, by halogen and/or alkyl having 1 or 2 carbon atoms,
$R^3$ represents straight-chain or branched alkyl having 1 to 10 carbon atoms, cycloalkyl having 5 to carbon atoms, and represents phenyl which is optionally substituted once or twice, identically or differently, by halogen and/or alkyl having 1 or 2 carbon atoms, or
$R^2$ and $R^3$, together with the carbon atom to which they are bonded, represent cycloalkyl having 5 to 7 carbon atoms, are used as starting materials.

4. Process according to Claim 1, characterized in that Lewis acids are used as acid catalysts.

5. Process according to Claim 1, characterized in that the reaction is carried out at temperatures between 50 and 100°C.

6. Process according to Claim 1, characterized in that 1 to 10 mol of ketoxime O-ether of the formula (III) and 0.01 to 10 mol-% of catalyst relative to ketoxime O-ether are used for 1 mol of hydroxybenzaldehyde of the formula (II).

**Revendications**

1. Procédé de préparation d'O-éthers d'hydroxy-benzald-oximes de formule

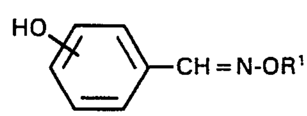 (I)

dans laquelle

$R^1$ représente un groupe alkyle, alcényle ou alcynyle,

procédé caractérisé en ce qu'on fait réagir, en présence d'un catalyseur acide et éventuellement en présence d'un diluant, à des températures comprises entre 50 et 150°C, des hydroxy-benzaldéhydes de formule

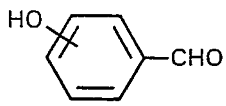
(II)

avec des O-éthers de cétoximes de formule

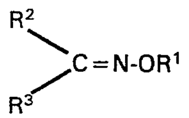
(III)

dans laquelle

$R^1$ a le sens indiqué ci-dessus, et
$R^2$ et $R^3$ sont identiques ou différents et représentent chacun un reste aliphatique, cycloaliphatique ou aromatique, ou bien
$R^2$ et $R^3$, pris avec l'atome de carbone auquel ils sont fixés, représentent un groupe cycloalkyle.

3. Procédé selon la revendication 1, caractérisé en ce qu'on utilise comme substance de départ de formule (II) le 4-hydroxybenzaldéhyde.

3. Procédé selon la revendication 1, caractérisé en ce qu'on utilise comme substances de départ des O-éthers de cétoximes de formule (III), dans lesquels

$R^1$ représente un groupe alkyle ou ramifié ayant 1 à 20 atomes de carbone, ainsi qu'un groupe alcényle et alcynyle, linéaires ou ramifiés, ayant chacun 3 à 10 atomes de carbone,
$R^2$ représente un groupe alkyle linéaire ou ramifié, ayant 1 à 10 atomes de carbone, un groupe cycloalkyle ayant 5 à 7 atomes de carbone, ainsi qu'un groupe phényle éventuellement substitué 1 ou 2 fois, de manière identique ou différente, par de l'halogène et/ou par un groupe alkyle ayant 1 ou 2 atomes de carbone,
$R^3$ représente un groupe alkyle linéaire ou ramifié, ayant 1 à 10 atomes de carbone, un groupe cycloalkyle ayant 5 à 7 atomes de carbone ainsi qu'un groupe phényle éventuellement substitué 1 ou 2 fois, de manière identique ou différente, par de l'halogène et/ou par un groupe alkyle ayant 1 ou 2 atomes de carbone, ou
$R^2$ ou $R^3$, pris avec l'atome de carbone auquel ils sont liés, représentent un groupe cycloalkyle ayant 5 à 7 atomes de carbone.

4. Procédé selon la revendication 1, caractérisé en ce qu'on utilise comme catalyseurs acides des acides de Lewis.

5. Procédé selon la revendication 1, caractérisé en ce qu'on conduit le réaction à des températures comprises entre 50 et 100°C.

6. Procédé selon la revendication 1, caractérisé en ce qu'on utilise, pour 1 mol de l'hydroxy-benzaldéhyde de formule (II) 1 à 10 mol de l'O-éther de cétoxime de formule (III) et 0,01 à 10 mol-% de catalyseur, sur la base de l'O-éther de cétoxime.